# EUROPEAN PATENT APPLICATION

(11) **EP 1 426 449 A1**
(43) Date of publication of application: **09.06.2004**
(21) Application number: 02755897.2
(22) Date of filing: 09.08.2002
(51) Int. Cl.: C12Q 1/68, A61K 45/00, A61P 35/00, G01N 33/50

(54) **METHOD OF EVALUATING DEGREE OF CANCERATION OF MAMMAL-ORIGIN SPECIMEN**

(30) Priority: 23.08.2001 JP 2001252804
(71) Applicant: Sumitomo Chemical Company, Limited, Osaka-shi Osaka 541-8550 (JP); JAPAN as represented by PRESIDENT OF NATIONAL CANCER CENTER, Chuo-ku, Tokyo 104-0045 (JP)
(72) Inventor: USHIJIMA, T. 1817,Nat. Cancer Tsukijilodging House, Tokyo 104-0045 (JP); MIYAMOTO, Kazuaki, Tokyo 110-0014 (JP)
(74) Representative: Duckworth, Timothy John
(86) International application number: PCT/JP2002/008161
(87) International publication number: WO 2003/018840

(57) **Abstract**

The present invention relates to a method for assessing a cancerous state of a mammal-derived specimen, which comprises:
(1) a first step of measuring a methylation frequency of Heparan sulfate D-glucosaminyl 3-0-sulfotransferase gene contained in a mammal-derived specimen or an index value having the correlation therewith, and
(2) a second step of determining a cancerous state of the specimen based on a difference obtained by comparing the measured methylation frequency or the index value having the correlation therewith, with a control; and the like.

SEQUENCE LISTING

<110> JAPAN as represented by PRESIDENT OF NATIONAL CANCER CENTER
<110> SUMITOMO CHEMICAL COMPANY, LIMITED

<120> METHOD OF EVALUATING DEGREE OF CANCERATION OF MAMMAL-ORIGIN
SPECIMEN

<130> N.91197

<140> EP 02755897.2
<141> 2002-08-09

<150> PCT/JP02/08161
<151> 2002-08-09

<150> JP 2000/252804
<151> 2001-08-23

<160> 21

<210> 1
<211> 1999
<212> DNA
<213> Homo sapiens

<400> 1
caaggtacca gtgtggtccc tcagggaagt actggggatc gtcacttatg cctgttctgg 60
acatggtcac cgagaactgt cctgtaggca ttcacttagg aatcattcga agtggaattg 120
ctcctggata cgttctcctt gtactctgtt tcctcctcct agtgtctctg tgtgaagaag 180
ccctcctcac tcagccctcg gcgaccctct ggtaccctgg acagctcccc ggggagcagt 240
ctaccgctag gcggcggctg ctaagagagg aaccctcctg acgcggagtc tgccgctccg 300
gggctcgctc tccggcaggc ccggggagag gtggggtgac aatgggttgg ggtgcgcgcg 360
tgcctcatag gtgcgagaca gagcgagccg ccggggtgtg agtcagcgcg ctgggggcta 420
agaagctggg tgaatagtca cggaatctca ctcacgctcg gctcctccac ccatcccgtc 480
tacagcgcgt gtcccagtcc agggcgtgcg tgcgctcggt gtccgattcc gggctgtgtg 540
tgtccatttg gcgagatgtc gagagcgggg ggagtgtcct tgtcggtgta tctgggccca 600
ggttagggga cttctcctcc ccacccccgc gtgggtgtgg gggtgtgtcc gggctagggc 660
gcgtgtgctt ctgtgcctgt gcgtgcgtgt gcgggtcagg gtggtgggac cgcgcatcag 720
ggcagggtgc ctgcgtctgc gtctgggtct gtctggtctg catgtcggcg cgatctcgac 780
ctggattcgt gtccctggat gtcgagaggc cagcgtggtg ggggtgtcca gcctcccgga 840
ggagtactat gccttgacac cttcgtttca ccgccccaaa gctggcctgg ggctccgtag 900
ggagtggcct gcatggggag ggcccgcgtg ctgtgtttct gggaggggta agagagtggg 960
ggcgcagggg gcgggccagg tccctgggcg cggcgcgggc tcgggggacc cgcgcggctg 1020
acgtcaggcc actccttaaa tagagccggc agcgcgctcc gctcggcatt tcccgaagag 1080
ccagatcgcg gccggcgcca gcgccaccgt ccggtccacc cgccagcccg cacagccgcg 1140
ccgccgccga gcgtttcgtg agcggcgctc cgaggatcag gaatggggct tcgggcgctg 1200
ggcgcgctcc gaacccggcg cacgtaagag cctgggagcg cccgagccgc ccggctgccc 1260
ggagccccat cgcctaggac cgggagatgc tggaaatgca acggcctgtt ccccgaggag 1320
ccgctgcccc cgggaccccc tggcactgtg cgcaccctgg tcagcagccc ccggagaaga 1380
cggcgccccc aacgcccgac ccgcgtggcc gtggcagcgc cacgcgagcc ctctaggcga 1440
ccgcagggcc acagcagctc agccgccggt gccccctcgg aaaccatgac ccccggcgcg 1500
ggcccatgga gccatggcct atagggtcct gggccgcgcg gggccacctc agccgcggag 1560
ggcgcgcagg ctgctcttcg ccttcacgct ctcgctctcc tgcacttacc tgtgttacag 1620
cttcctgtgc tgctgcgacg acctgggtcg gagccgcctc ctcggcgcgc ctcgctgcct 1680
ccgcggcccc agcgcgggcg gccagaaact tctccagaag tcccgcccct gtgatccctc 1740
cgggccgacg cccagcgagc ccagcgctcc cagcgcgccc gccgccgccg tgcccgcccc 1800
tcgcctctcc ggttccaacc actccggctc acccaagctg ggtaccaagc ggttgcccca 1860
agccctcatt gtgggcgtga agaagggggg cacccgggcc gtgctggagt ttatccgagt 1920
acacccggac gtgcgggcct tgggcacgga accccacttc tttgacagga actacggccg 1980
cgggctggat tggtacagg 1999

<210> 2
<211> 21
<212> DNA
<213> Artificial Sequence

<220>
<221> source
<223> Designed oligonucleotide primer for PCR

<400> 2
tgtgttttga atttggtgta t     21

<210> 3
<211> 23
<212> DNA
<213> Artificial Sequence

<220>
<221> source
<223> Designed oligonucleotide primer for PCR

<400> 3
aaaactcaca taacactacc aca     23

<210> 4
<211> 22
<212> DNA
<213> Artificial Sequence

<220>
<221> source
<223> Designed oligonucleotide primer for PCR

<400> 4
tggagtttta ttgtttagga tt     22

<210> 5
<211> 22
<212> DNA
<213> Artificial Sequence

<220>
<221> source
<223> Designed oligonucleotide primer for PCR

<400> 5
aaatcaaaca ttaaaaacac ca     22

<210> 6
<211> 19
<212> DNA
<213> Artificial Sequence

<220>
<221> source
<223> Designed oligonucleotide primer for PCR

<400> 6
cgtttcgaat tcggcgtac     19

<210> 7
<211> 20
<212> DNA
<213> Artificial Sequence

<220>
<221> source
<223> Designed oligonucleotide primer for PCR

<400> 7
cgaatcgaac gttaaaaacg     20

<210> 8
<211> 20
<212> DNA
<213> Artificial Sequence

<220>
<221> source
<223> Designed oligonucleotide primer for PCR

<400> 8
cggttgttcg gagttttatc     20

<210> 9
<211> 20
<212> DNA
<213> Artificial Sequence

<220>
<221> source
<223> Designed oligonucleotide primer for PCR

<400> 9
gtaacgctac cacgaccacg     20

<210> 10
<211> 21
<212> DNA
<213> Artificial Sequence

<220>
<221> source
<223> Designed oligonucleotide primer for PCR

<400> 10
gtgtatgtaa gagtttggga g     21

<210> 11
<211> 21
<212> DNA
<213> Artificial Sequence

<220>
<221> source
<223> Designed oligonucleotide primer for PCR

<400> 11
gcgtacgtaa gagtttggga g     21

<210> 12

<211> 25
<212> DNA
<213> Artificial Sequence

<220>
<221> source
<223> Designed oligonucleotide primer for PCR

<400> 12
ctccaaaaac tactaaccaa aatac     25

<210> 13
<211> 25
<212> DNA
<213> Artificial Sequence

<220>
<221> source
<223> Designed oligonucleotide primer for PCR

<400> 13
ctccgaaaac tactaaccaa aatac     25

<210> 14
<211> 19
<212> DNA
<213> Artificial Sequence

<220>
<221> source
<223> Designed oligonucleotide for probe

<400> 14
tgttttgaat ttggtgtat     19

<210> 15
<211> 19
<212> DNA
<213> Artificial Sequence

<220>
<221> source
<223> Designed oligonucleotide for probe

<400> 15
cgtttcgaat tcggcgtac     19

<210> 16
<211> 21
<212> DNA
<213> Artificial Sequence

<220>
<221> source
<223> Designed oligonucleotide for probe

<400> 16
ttgggatttt ttggtattgt g     21

<210> 17
<211> 21

<212> DNA
<213> Artificial Sequence

<220>
<221> source
<223> Designed oligonucleotide for probe

<400> 17
tcgggatttt ttggtattgt g     21

<210> 18
<211> 21
<212> DNA
<213> Artificial Sequence

<220>
<221> source
<223> Designed oligonucleotide primer for PCR

<400> 18
cgtgctggag tttatccgag t     21

<210> 19
<211> 21
<212> DNA
<213> Artificial Sequence

<220>
<221> source
<223> Designed oligonucleotide primer for PCR

<400> 19
caaagtagct gggcgtcttc t     21

<210> 20
<211> 21
<212> DNA
<213> Artificial Sequence

<220>
<221> source
<223> Designed oligonucleotide primer for PCR

<400> 20
aggtgaaggt cggagtcaac g     21

<210> 21
<211> 20
<212> DNA
<213> Artificial Sequence

<220>
<221> source
<223> Designed oligonucleotide primer for PCR

<400> 21
aggggtcatt gatggcaaca     20

## Description

### TECHNICAL FIELD

The present invention relates to a method for assessing a cancerous state of a mammal-derived specimen, and the like.

### BACKGROUND ART

Although it has been gradually revealed that a cancer is a disease, a cause of which is gene abnormality, the mortality of cancer patients is still high, demonstrating that an assessment of a diagnosing method and a treating method which are currently available are not necessarily fully satisfactory. One of causes therefor is considered to be variety based on a kind of cancer tissues, low correctness and low detection sensitivity of genes as a marker, and the like.

Then, there is desired development of a method for assessing a cancerous state of a mammal-derived specimen based on detection of a gene abnormality, which is suitable to assess such as a diagnosing method and a treating method for early finding a cancer.

### DISCLOSURE OF THE INVENTION

Under such the circumstances, the present inventors intensively studied and, as a result, have found that a Heparan sulfate D-glucosaminyl 3-0-sulfotransferase-2 gene (hereinafter, referred to as 3OST2 gene in some cases) is methylated in a cancer cell line at a significantly higher frequency as compared with a tissue specimen of a healthy subject and, in this cancer cell line, the expression level of 3OST2 gene is significantly lower as compared with a tissue specimen of a healthy subject and, further, have found that the expression level of such the gene can be increased by acting a DNA methylation inhibitor on the cancer cell line, which resulted in completion of the present invention.

That is, the present invention provides:
1. a method for assessing a cancerous state of a mammal-derived specimen, which comprises :
   (1) a first step of measuring a methylation frequency of Heparan sulfate D-glucosaminyl 3-0-sulfotransferase gene contained in a mammal-derived specimen or an index value having the correlation therewith, and
   (2) a second step of determining a cancerous state of the specimen based on a difference obtained by comparing the measured methylation frequency or the index value having the correlation therewith, with a control (hereinafter, referred to as present assessing method in some cases);
2. the assessing method according to the above 1, wherein Heparan sulfate D-glucosaminyl 3-0-sulfotransferase gene is Heparan sulfate D-glucosaminyl 3-0-sulfotransferase-2 gene;
3. the assessing method according to the above 1, wherein the mammal-derived specimen is cells;
4. the assessing method according to the above 1, wherein the mammal-derived specimen is a tissue;
5. a method for assessing a cancerous state of a mammal-derived specimen, which comprises:
   (1) a first step of measuring a methylation frequency of Heparan sulfate D-glucosaminyl 3-0-sulfotransferase gene contained in the mammal-derived specimen, and
   (2) a second step of determining a cancerous state of the specimen based on a difference obtained by comparing the measured methylation frequency with a control;
6. the assessing method according to the above 5, wherein Heparan sulfate D-glucosaminyl 3-0-sulfotransferase gene is Heparan sulfate D-glucosaminyl 3-0-sulfotransferase-2 gene;
7. the assessing method according to the above 1, wherein the mammal-derived specimen is cells, and the cancerous state of the specimen is a malignancy of mammal-derived cells ;
8. the assessing method according to the above 6, wherein the mammal-derived specimen is cells, and the cancerous state of the specimen is a malignancy of a mammal-derived cell;
9. the assessing method according to the above 1, wherein the mammal-derived specimen is a tissue, and the cancerous state of the specimen is an amount of cancer cells existing in a mammal-derived tissue;
10. the assessing method according to the above 6, wherein the mammal-derived specimen is a tissue, and the cancerous state of the specimen is an amount of cancer cells existing in a mammal-derived tissue;
11. the assessingmethod according to the above 10, wherein the tissue is a breast tissue, a mammary gland tissue or a mammary gland epithelial tissue, and the cancer is breast cancer;
12. the assessing method according to the above 1 or 6, wherein the methylation frequency of a gene is a methylation frequency of cytosine in one or more nucleotide sequence(s) represented by 5'-CG-3' present in a nucleotide sequence of a promoter region or a coding region of the gene;
13. the assessing method according to the above 12, wherein the tissue is a breast tissue, a mammary gland tissue or a mammary gland epithelial tissue, and the cancer is breast cancer;
14. the assessing method according to the above 1 or 6, wherein the methylation frequency of a gene is a methylation frequency of cytosine in one or more nucleotide sequence(s) represented by 5'-CG-3' present in a nucleotide sequence of a promoter region in the gene;
15. the assessing method according to the above 1 or 6, wherein the methylation frequency of a gene is a methylation frequency of cytosine in one or more nucleotide sequence(s) represented by 5'-CG-3' present in a nucleotide sequence of a coding region of the gene;
16. the assessing method according to the above 1, wherein the methylation frequency of a gene is a methylation frequency of cytosine in one or more nucleotide sequence(s) represented by 5'-CG-3' present in the nucleotide sequence represented by SEQ ID No: 1;
17. the assessing method according to the above 16, wherein the tissue is breast tissue, mammary gland tissue or mammary gland epithelial tissue, and the cancer is breast cancer;
18. a method for assessing a cancerous state of a mammal derived specimen, which comprises :
   (1) a first step of measuring an index value having the correlation with a methylation frequency of Heparan sulfate D-glucosaminyl 3-0-sulfotransferase gene contained in the mammal-derived specimen, and
   (2) a second step of determining a cancerous state of the specimen based on a difference obtained by comparing the index value having the correlation with the measured methylation frequency with a control ;
19. the assessing method according to the above 18, wherein Heparan sulfate D-glucosaminyl 3-0-sulfotransferase gene is Heparan sulfate D-glucosaminyl 3-0-sulfotransferase-2 gene;
20. the assessing method according to the above 18, wherein the index value having the correlation with a methylation frequency of Heparan sulfate D-glucosaminyl 3-0-sulfotransferase gene is an amount of an expression product of the Heparan sulfate D-glucosaminyl 3-0-sulfotransferase gene;
21. the assessingmethod according to the above 19, wherein the index value having the correlation with a methylation frequency of Heparan sulfate D-glucosaminyl 3-0-sulfotransferase gene is an amount of an expression product of the Heparan sulfate D-glucosaminyl 3-0-sulfotransferase gene;
22. the assessing method according to the above 20 or 21, wherein the amount of an expression product of Heparan sulfate D-glucosaminyl 3-0-sulfotransferase gene is an amount of a transcription product of the gene;
23. the assessing method according to the above 20 or 21, wherein the amount of an expression product of Heparan sulfate D-glucosaminyl 3-0-sulfotransferase gene is an amount of a translation product of the gene;
24. a method for searching a substance having the ability of promoting the expression of Heparan sulfate D-glucosaminyl 3-0-sulfotransferase gene, which comprises:
   (1) a first step of bringing a test substance into contact with a cancer cell,
   (2) a second step of measuring an amount of an expression product of 3OST gene contained in the cancer cell after the first step (1), and
   (3) a third step of determining the ability of the test substance to promote the expression of Heparan sulfate D-glucosaminyl 3-0-sulfotransferase gene possessed by , based on a difference obtained by comparing the measured amount of an expression product with a control (hereinafter, referred to as present searching method in some cases);
25. the searching method according to the above 24, wherein Heparan sulfate D-glucosaminyl 3-0-sulfotransferase gene is Heparan sulfate D-glucosaminyl 3-0-sulfotransferase-2 gene ;
26. the searching method according to the above 24, wherein the cancer cell is breast cancer cell;
27. the searching method according to the above 25, wherein the cancer cell is breast cancer cell;
28. an anti-cancer agent, which comprises a substance having the ability found by the searching method of the above 24 as an active ingredient, wherein the active ingredient is formulated into a pharmaceutically acceptable carrier;
29. an anti-cancer agent, which comprises a nucleic acid comprising a nucleotide sequence encoding an amino acid sequence of Heparan sulfate D-glucosaminyl 3-0-sulfotransferase as an active ingredient, wherein the active ingredient is formulated into a pharmaceutically acceptable carrier;
30. use of methylated Heparan sulfate D-glucosaminyl 3-0-sulfotransferase gene as a cancer marker;
31. the use according to the above 30, wherein the cancer marker is a breast cancer marker;
32. use of a methylated Heparan sulfate D-glucosaminyl 3-0-sulfotransferase-2 gene as a cancer marker;
33. the use according to the above 32, wherein the cancer marker is a breast cancer marker;
34. a method for inhibiting canceration, which comprises a step of administering a substance which reduces a methylation frequency of Heparan sulfate D-glucosaminyl 3-0-sulfotransferase gene, to cells in a body of a mammal which can be diagnosed as a cancer;
35. the canceration inhibiting method according to the above 34, wherein Heparan sulfate D-glucosaminyl 3-0-sulfotransferase gene is a Heparan sulfate D-glucosaminyl 3-0-sulfotransferase-2 gene;
36. the canceration inhibiting method according to the above 35, wherein the cancer is breast cancer ;
37. the assessing method according to the above 1, wherein the mammal-derived specimen is blood derived from a human being who is under 55 years old.

### BRIEF DESCRIPTIONS OF THE DRAWINGS

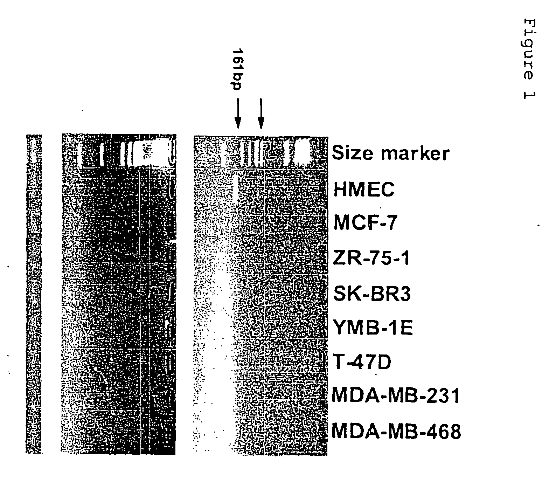

Figure 1 is a view (photograph) showing the results obtained by analyzing with agarose gel electrophoresis amplification products obtained by PCR amplifying a DNA (161 bp) derived from a mRNA of 3OST2 gene, from human-derived normal mammary gland epithelial cell (HMEC) and seven kinds of breast cancer cell lines. Names of cells used are shown above the view (photograph). The view (photograph) at an upper step shows the results of PCR performed by using a cDNA prepared from each cell as a template, and using primers 3OST2 S and 3OST2 A. The view (photograph) at a middle step shows the results of PCR performed by using a RNA prepared from each cell as a template, and using primers 3OST2 S and 3OST2 A. The view (photograph) at a lower step shows the results of PCR performed by using a cDNA prepared from each cell as a template, and using primers GAPDH S and GAPDH A.

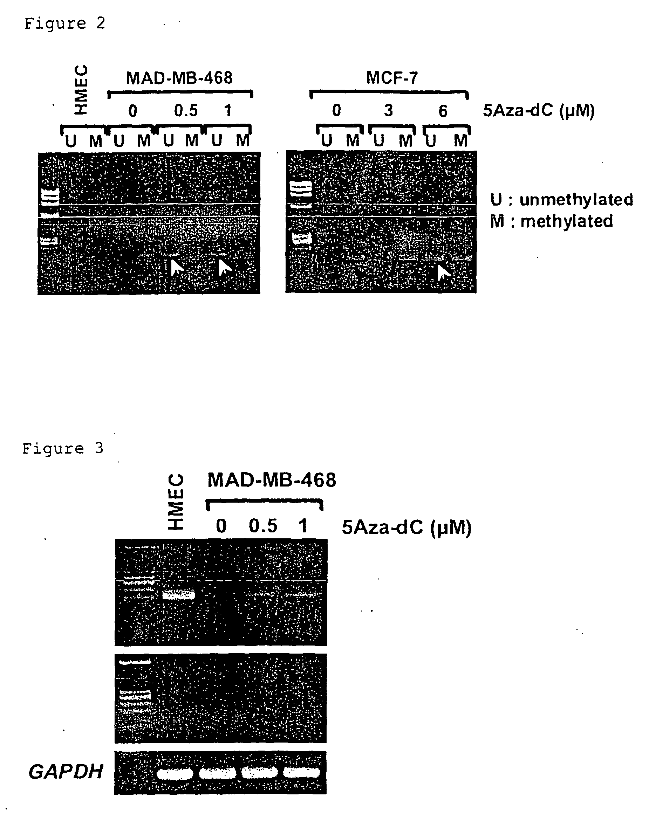

Figure 2 is a view (photograph) showing the results obtained by performing PCR using, as a template, genomic DNAs prepared from human-derived normal mammary gland epithelial cell (HMEC) and two kinds of breast cancer cell lines and treated with sodium bisulfite, respectively, and analyzing the PCR reaction solutions after PCR with agarose gel electrophoresis. Names of cells used and the concentration (µM) of 5Aza-dC added upon culturing of the cells are shown above the view (photograph). Lane U (Unmethylated) indicates the case of the PCR reaction solution of PCR using a non-methylated specific primer, and lane M (Methylated) indicates the case of the PCR reaction solution of PCR using a methylation-specific primer.
Figure 3 is a view (photograph) showing the results obtained by analyzing, with agarose gel electrophoresis, an amplification product obtained by PCR amplifying a DNA (161 bp) derived from a mRNA of 3OST2 gene with PCR, from human-derived normal mammary gland epithelial cell (HMEC) and a breast cancer cell line MDA-MB-468. Names of cells used and the concentration (µM) of 5 Aza-dC added upon culturing of the cells are shown above the view (photograph). The view (photograph) at an upper step shows the results obtained by performing PCR using a cDNA prepared from each cell as a template, and using primers 3OST2 S and 3OST 2 A. A view (photograph) at a middle step shows the results obtained by performing PCR using a RNA prepared from each cell as a template, and using primers 3OST2 S and 3OST2 A. A view (photograph) at a lower step shows the results obtained byperforming PCR using a cDNAprepared from each cell as a template, and using primers GAPDH S and GAPDH A.

### BEST MODE FOR CARRYING OUT THE INVENTION

The present invention will be explained in detail below.

The present invention relates to use of methylated Heparan sulfate D-glucosaminyl 3-0-sulfotransferase gene (hereinafter, referred to as 3OST gene in some cases) (e.g. 3OST2 gene or the like) as a cancer marker (e.g. mammary cancer marker or the like), and the like.

Examples of 3OST gene (such as 3OST2 gene) used as a marker gene in the present invention include a gene containing a coding region containing a nucleotide sequence encoding an amino acid sequence of human-derived 3 OST gene and a promoter region situated on a 5' upstream side thereof. Embodiments thereof include a gene containing a coding region containing a nucleotide sequence encoding an amino acid sequence of human-derived Heparan sulfate D-glucosaminyl 3-0-sulfotransferase -2 gene (hereinafter, referred to as 3OST2 gene in some cases) [J. Biol. Chem., 274, 5170-5184 (1999)] and a promoter region situated on a 5' upstream side thereof. An amino acid sequence of human-derived 3OST2 gene and a nucleotide sequence encoding it are described, for example, in Genbank Accession No. AF105375 and the like. A nucleotide sequence of a genomic DNA, containing an exon situated on a 5' most-upstream side (hereinafter, referred to as exon 1) among a coding region containing a nucleotide sequence encoding an amino acid sequence of human-derived 3OST2 gene and a promoter region situated on a 5' upstream side thereof, is described, for example, in Genbank Accession No. HUAC003661 and the like. In a nucleotide sequence described in Genbank Accession No. HUAC003661 and the like, for example, an ATG codon encoding methionine situated at a terminal of an amino acid sequence of human-derived 30TS2 protein is shown by nucleotide numbers 58514 to 58516, and a nucleotide sequence of the exon 1 is shown by nucleotide numbers 58514 to 58999. The 3OST gene such as a 30TS2 gene utili zed in the present invention includes, in addition to the aforementioned gene having the known nucleotide sequence, a gene having a nucleotide sequence such as deletion, substitution or addition of a nucleotide has occurred in such the nucleotide sequence, deriving from a naturally occurring mutation due to a difference in organism species, a difference between individuals, or a difference between organs or tissues, or the like.

There is the phenomenon that, among four kinds of bases constituting a gene (genomic DNA), only cytosine is methylated in mammals. For example, in mammal-derived 3OST gene such as 3OST2 gene, some of cytosines of a genomic DNA of the gene are methylated. And, methylation modification of DNA is limited to cytosines in a nucleotide sequence represented by 5'-CG-3' (C represents cytosine, and G represents guanine; hereinafter, the nucleotide sequence is referred to as CpG in some cases). In cytosine, a site to be methylated is a 5-position thereof. Upon DNA replication prior to cell division, only cytosine in CpG of a template chain is methylated immediately after replication, but cytosine in CpG of a newly produced chain is also quickly methylated by the action of methyltransferase. Therefore, the status of methylation of DNA is inherited as it is to new two sets of DNAs also after DNA replication.

In the first step of the present assessing method, a "methylation frequency" is represented, for example, by a ratio of haploids in which the cytosine is methylated, when the presence or the absence of methylation of cytosine in CpG to be investigated, is investigated for plural haploids.

Further, in the first step of present assessing method, examples of an "index value having the correlation with a (methylation frequency)" include an amount of an expression product of 3OST gene (more specifically, an amount of a transcription product of the gene, and an amount of a translation product of the gene) and the like. In the case of such the amount of an expression product, there is such the negative correlation that as the methylation frequency grows higher, the amount decreases accordingly.

Examples of the mammal-derived specimen in the first step of the present assessing method include living body samples such as cancer cells such as breast cancer cells or a tissue containing it, and cells potentially containing a DNA derived from cancer cells such as breast cancer cells, a tissue containing it (herein, a tissue broadlymeans including body fluids such as blood, plasma, serum, lymph and the like; lymph node and the like) or living body secreted substances (urine, milk and the like). Specifically, for example, when the cancer is breast cancer, examples include breast tissue, mammary gland tissue or mammary gland epithelial tissue taken from a subject animal.

These living body samples may be used as it is as a specimen, or living body samples prepared by various procedures such as separation, fractionation, immobilization and the like from such the living body samples may be used as a specimen.

When the mammal-derived specimen is blood, the present assessing method can be expected to be utilized in periodic physical checking, simple test, and the like. In this case, in order to effectively utilize the present assessing method while suppressing a false determination rate low, blood derived from a human being under 55 years old is preferable.

In the first step of the present assessing method, a method for measuring a methylation frequency of 3OST gene contained in a mammal-derived specimen or an index value having the correlation therewith may be performed, for example, as follows:

As a first method, a DNA is first extracted from a mammal-derived specimen, for example, using a commercially available DNA extracting kit or the like.

Incidentally, when blood is used as a specimen, plasma or serum is prepared from the blood according to the conventional method, and free DNA (including a DNA derived from cancer cells such as breast cancer cells and the like) contained in the prepared plasma or serum as a specimen is analyzed, whereby, a DNA derived from cancer cells such as breast cancer cell can be analyzed while avoiding a hemocyte-derived DNA, and a sensitivity for detecting cancer cell such as breast cancer cell, or a tissue containing it can be improved.

Then, after the extracted DNA is contacted with a reagent which modifies unmethylated cytosine, a DNA containing cytosine in one or more nucleotide sequence (s) represented by CpG which is present in a nucleotide sequence of a promoter region or a coding region of 3OST gene is amplified by a polymerase chain reaction (hereinafter, referred to as PCR) using primers which can recognize the presence or the absence of methylation of cytosine to be analyzed, and an amount of the resulting amplification product is investigated.

Herein, for example, when 3OST gene is 3OST2 gene, examples of one or more nucleotide sequence (s) represented by CpG which is present in a nucleotide sequence of a promoter region or a coding region of 3OST gene include a nucleotide sequence of a genomic DNA containing an exon 1 of human-derived 3OST2 gene and a promoter region situated on a 5' upstream side thereof, more specifically, the nucleotide sequence represented by SEQ ID NO: 1 (corresponding to the nucleotide sequence represented by nucleotide numbers 57001 to 58999 of a nucleotide sequence described in Genbank Accession No. HUAC003661). In the nucleotide sequence represented by SEQ ID NO: 1, the ATG codon encoding methionine at the amino terminal of human-derived 3OST2 protein is shown by nucleotide numbers 1514 to 1516, and the nucleotide sequence of the exon 1 is shown by nucleotide numbers 1514 to 1999. Cytosine in a nucleotide sequence represented by CpG which is present in the nucleotide sequence represented by SEQ ID NO: 1, inter alia, cytosine in CpG present in a region in which CpGs are densely present in the nucleotide sequence represented by SEQ ID NO: 1 shows a high methylation frequency (i.e. hypermethylation), for example, in cancer cells such as breast cancer cells. More specifically, examples of cytosine having a high methylation frequency in a breast cancer cell include cytosines represented by nucleotide numbers 1239, 1243, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence of SEQ ID NO: 1.

As a reagent for modifying unmethylated cytosine, for example, bisulfite such as sodium bisulfite can be used.

In order that the extracted DNA is contacted with a reagent for modifying unmethylated cytosine, for example, the DNA is first treated with bisulfite such as sodium bisulfite (concentration in a solution: e.g. final concentration 3M) at 55°C for around about 10 to 16 hours (overnight) in an alkaline solution (pH 9 to 14). In this case, unmethylated cytosine is converted into uracil and, on the other hand, methylated cytosine is not converted into uracil, but still remains as cytosine.

Then, PCR using a DNA treated with bisulfite or the like as a template, and using one pair of methylation-specific primers, each selected from a nucleotide sequence whenmethylated cytosine is contained in the nucleotide sequence in which cytosine at a position to be methylated (cytosine in CpG) still remains as cytosine, and unmethylated cytosine (cytosine not contained in CpG) is converted into uracil and a nucleotide sequence complementary to such the nucleotide sequence (hereinafter, also referred to as methylation-specific PCR in some cases) , and PCR using a DNA treated with bisulfite as a template, and using one pair of unmethylation-specific primers, each selected from a nucleotide sequence when cytosine is not methylated (nucleotide sequence in which all cytosines are converted into uracil) and a nucleotide sequence complementary to such the nucleotide sequence (hereinafter, also referred to as unmethylation-specific PCR in some cases) are performed.

In the aforementioned PCR, in the case of PCR using the methylation-specific primer (former), a DNA in which cytosine to be analyzed is methylated is amplified and, on the other hand, in the case of PCR using the unmethylation-specific primer (latter) , a DNA in which cytosine to be analyzed is not methylated is amplified. By comparing amounts of these amplification products, the presence or the absence of methylation of cytosine to be analyzed is investigated. Like this, a methylation frequency can be measured.

Herein, inviewofthat, inthemethylation-specificprimer, cytosine which has not undergone methylation is converted into uracil, and cytosine which has undergone methylation is not converted into uracil, a PCR primer specific for a nucleotide sequence containing cytosine which has undergone methylation (methylation-specific primer) is designed, and a PCR primer specific for a nucleotide sequence containing cytosine which has not undergone methylation (unmethylation-specific primer) is designed. Since design is performed based on a DNA chain which has been chemically converted by sulfite treatment and has become not complementary, based on respective chains of DNAs which were originally double-stranded, a methylation specific primer and a unmethylation-specific primer may be also prepared from respective chains. In order to enhance specificity for methyl or non-methyl, such the primers are preferably designed so that primers contain cytosine in CpG near a 3'-terminal of primers. Moreover, in order tomake analysis easy, one of primers may be labeled.

More specifically, when 3OST gene is 3OST2 gene, a primer for measuring a methylation frequency of the gene with methylation-specific PCR can be designed as described above, for example, based on a nucleotide sequence containing one or more cytosine(s) in CpG present in a nucleotide sequence in a promoter region or a coding region of 3OST2 gene. For example, design can be performed based on a nucleotide sequence containing one or more cytosine (s) in CpG present in a region in which CpGs are densely present in the nucleotide sequence represented by SEQ ID NO: 1, more specifically, cytosine(s) represented by nucleotide numbers 1239, 1243, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence represented by SEQ ID NO: 1. Examples of such the primers are shown below.

### <Unmethylation -specific primer>

U1: 5'-TGTGTTTTGAATTTGGTGTAT-3' (SEQ ID No: 2)

U2: 5'-AAAACTCACATAACACTACCACA-3' (SEQ ID No: 3)

U3: 5'-TGGAGTTTTATTGTTTAGGATT-3' (SEQ ID No: 4)

U4: 5'-AAATCAAACATTAAAAACACCA-3' (SEQ ID No: 5)

### <Methylation-specific primer>

M1: 5'-CGTTTCGAATTCGGCGTAC-3' (SEQ ID No: 6)

M2: 5'-CGAATCGAACGTTAAAAACG-3' (SEQ ID No: 7)

M3: 5'-CGGTTGTTCGGAGTTTTATC-3' (SEQ ID No: 8)

M4: 5'-GTAACGCTACCACGACCACG-3' (SEQ ID No: 9)

### <Combination of primers>

Combination 1: U1-U2, M1-M4
Combination 2: U1-U4, M1-M2
Combination 3: U3-U2, M3-M4
Combination 4: U3-U4, M3-M2

Examples of a reaction solution in the methylation-specific PCR include a reaction solution obtained by mixing 20 ng of a DNA to be a template, each 1 µl of 30 pmol/µl of each primer solution, 3 µl of 2 mM dNTP, 3 µl of 10 × buffer (100 mM Tris-HCl pH 8.3, 500 mM KCl, 20 mM MgCl₂), and 0.2 µl of 5U/µl thermostable DNA polymerase, and adding sterilized ultrapure water to an amount of 30 µl. Examples of reaction conditions include the condition under which the aforementioned reaction solution is retained at 94°C for 10 minutes and, thereafter, 40 cycles of temperature maintenance is performed, each cycle being 30 seconds at 94°C, then 60 seconds at 55 to 65°C and 45 seconds at 72°C.

After such the PCR is performed, amounts of the resulting amplification products are compared. For example, in the case of an analyzing method which can compare amounts of respective amplification products obtained by PCR using the methylation-specific primer and PCR using the unmethylation-specific primer (denatured polyacrylamide gel electrophoresis or agarose gel electrophoresis), a gel after electrophoresis is put on DNA-staining to detect bands of amplification products, and density of the detected bands are compared. Herein, using pre-labeled primers in place of DNA-staining, the density of bands may be compared using the label as an index.

Such the method is the method which is generally also called methylation-specific PCR and was reported by Herman (Herman et al., Proc. Natl. Acad. Sci USA, 93, 9821-9826, 1996), and this method utilizes a difference in the chemical property between cytosine and 5-methylcytosine.

As a second method, a DNA is first extracted from a mammal-derived specimen, for example, using a commercially available DNA extracting kit or the like.

Incidentally, when blood is used as a specimen, plasma or serum is prepared from the blood according to the conventional method, and a free DNA (including a DNA derived from cancer cells such as breast cancer cells) contained in the prepared plasma or serum as a specimen is analyzed, thereby, a DNA derived from cancer cells such as breast cancer cells can be analyzed while avoiding a hemocyte-derived DNA, and a sensitivity for detecting cancer cells such as breast cancer cells, or a tissue or the like containing it can be improved.

There is also a method in which, then, the extracted DNA is contacted with a reagent for modifying unmethylated cytosine, and amplified by a polymerase chain reaction (hereinafter, referred to as PCR) using primers which are designed as described below based on a DNA containing cytosine in one or more nucleotide sequence (s) represented by CpG which is present in a nucleotide sequence of a promoter region or a coding region of 3OST gene, and a nucleotide sequence of the resulting amplification product is directly analyzed.

Herein, when 3OST gene is 3OST2 gene, examples of one or more nucleotide sequence(s) represented by CpG present in a nucleotide sequence of a promoter region or a coding region of 3OST gene include a nucleotide sequence of a genomic DNA containing an exon 1 of human-derived 3OST2 gene and a promoter region situated on a 5' upstream side thereof, more specifically, the nucleotide sequence represented by SEQ ID NO: 1 (corresponding to the nucleotide sequence represented by nucleotide numbers 57001 to 58999 of the nucleotide sequence described in Genbank Accession No. HUAC003661). In the nucleotide sequence represented by SEQ ID NO: 1, the ATG codon encoding methionine at the amino-terminalofhuman-derived 3OST2 protein is shown by nucleotide numbers 1514 to 1516, and the nucleotide sequence of the aforementioned exon 1 is shown by nucleotide numbers 1514 to 18999. Cytosine in a nucleotide sequence represented by CpG present in the nucleotide sequence represented by SEQ ID NO: 1, inter alia, cytosine in CpG present in a region in which CpGs are densely present in the nucleotide sequence represented by SEQ ID NO: 1 shows a high methylation frequency (i.e. hypermethylation status) in cancer cells such as breast cancer cells. More specifically, examples of cytosine having a high methylation frequency in a breast cancer cell include cytosines represented by nucleotide numbers 1239, 1243, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence represented by SEQ ID NO: 1.

As a primer used in the PCR, it is better to design a pair of primers which can amplify a DNA having a nucleotide sequence containing the cytosine, based on a nucleotide sequence of a 5' upstream side of cytosine to be analyzed and a nucleotide sequence of 3' downstream side thereof. A nucleotide sequence for primer design is selected so that it does not contain cytosine in CpG to be analyzed. And, when a nucleotide sequence selected for primer design does not contain cytosine at all, a selected nucleotides sequence and a nucleotide sequence complementary to such the nucleotide sequence can be employed as they are, respectively, as a nucleotide sequence for a primer. In addition, when a nucleotide sequence selected for primer design contains cytosine other than that to be analyzed, but the cytosine is not cytosine in CpG, a primer is designed in view of that these cytosines are converted into uracil. That is, one pair of primers, each having a nucleotide sequence in which all cytosines are converted into uracil and a nucleotide sequence complementary to such the nucleotide sequence is designed. Further, when a nucleotide sequence selected for primer design contains cytosine other than that to be analyzed, and the cytosine is cytosine in CpG, primers are designed in view of that cytosine which has not undergone methylation is converted into uracil, and cytosine which has undergone methylation is not converted into uracil. That is, one pair of methylation specific primers, respectively, selected from a nucleotide sequence which contains methylated cytosine [a nucleotide sequence in which cytosine at a position to be methylated (cytosine in CpG) still remains as cytosine, and unmethylated cytosine (cytosine not contained in CpG) is converted into uracil] and a nucleotide sequence complementary to such the nucleotide sequence, and one pair of unmathylation-specific primers, each having a nucleotide sequence in which cytosine is not methylated (a nucleotide sequence in which all cytosines are converted into uracils) and a nucleotide sequence complementary to such the nucleotide sequence are designed. In this case, equivalent amounts of the methylation-specific primer pair and the unmethylation-specific primer pair are used in the aforementioned PCR, by mixing them.

As a reagent for modifying unmethylated cytosine, bisulfite such as sodium bisulfite can be used.

In order that the extracted DNA is contacted with a reagent for modifying unmethylated cytosine, for example, the DNA is first treated with bisulfite such as sodium bisulfite (concentration in a solution: for example, the final concentration 3M) at 55°C for around about 10 to 16 hours (overnight) in an alkaline solution (pH 9 to 14). In this case, unmethylated cytosine is converted into uracil and, on the other hand, methylated cytosine is not converted into uracil, and still remains as cytosine.

Then, PCR is performed using a DNA treated with bisulfite or the like as a template, and using primers which are designed as described above. Nucleotide sequences of the resulting amplification products are compared, andamethylation frequency can be measured by the comparison.

More specifically, when 3OST gene is 3OST2 gene, primers for measuring a methylation frequency of the gene by direct analysis of a nucleotide sequence can be designed as described above, for example, based on a nucleotide sequence containing one or more cytosine(s) in CpG present in a nucleotide sequence which is in a promoter region or a coding region of the 3OST2 gene. For example, primers can be designed based on a nucleotide sequence containing one or more cytosine(s) in CpG present in a region in which CpGs are densely present in the nucleotide sequence represented by SEQ ID NO: 1, specifically, cytosine (s) represented by nucleotide numbers 1239, 1234, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence represented by SEQ ID NO: 1. Examples of such the primers are shown below.

Incidentally, when the methylation-specific primer pair and the unmethylation-specific primer pair designed as described above are used in order to investigate a methylation frequency of cytosines represented by nucleotide numbers 1239, 1243, 1248, 1252, 1260, 1271, 1281 and 1303 in the nucleotide sequence represented by SEQ ID NO: 1, a DNA (159 bp) containing the nucleotide sequence represented by nucleotide numbers 1218 to 1376 in the nucleotide sequence represented by SEQ ID NO: 1 is amplified.

### <Mixed primer>

BU1: 5'-GTGTATGTAAGAGTTTGGGAG-3' (unmethylation-specific
primer)(SEQ ID NO: 10)

BM1: 5'-GCGTACGTAAGAGTTTGGGAG-3' (methylation-specific
primer)(SEQ ID NO: 11)

BU2: 5'-CTCCAAAAACTACTAACCAAAATAC-3' (unmethylation-specific

primer)(SEQ ID NO: 12)

BM2: 5'-CTCCGAAAACTACTAACCAAAATAC-3' (methylation specific
primer)(SEQ ID NO: 13)

Examples of a reaction solution in PCR include a reaction solution obtained by mixing 20 ng of a DNA as a template, each 1 µl of four kinds of 30 pmol/µl respective primer solutions, 3 µl of 2 mM dNTP, 3 µl of 10 × buffer (100 mM Tris-HCl pH 8.3, 500 mM KCl, 20 mM MgCl₂) and 0.2 µl of 5U/µl thermostable DNA polymerase, and adding sterilized ultrapure water to this to an amount of 30 µl. Examples of the reaction conditions include the condition in which the aforementioned reaction solution is retained at 94°C for 10 minutes and, thereafter, 40 cycles of temperature maintenance is performed, one cycle being 30 seconds at 94°C, then 60 seconds at 55°C, and further 45 seconds at 72°C.

After such the PCR is performed, nucleotide sequences of the resulting amplification products are compared, and a methylation freqeuncy is measured from the comparison.

That is, by directly analyzing nucleotide sequences of the amplification products, whether a base at a position corresponding to cytosine to be analyzed is cytosine or thymine (uracil) is determined. By comparing an area of a peak showing cytosine and an area of a peak showing thymine (uracil) both detected at a position corresponding to cytosine to be analyzed in a chart of peaks showing nucleotides in the resulting amplification products, a methylation frequency of cytosine to be analyzed can be measured. Alternatively, as a method for directly analyzing a nucleotide sequence, after the amplification products obtained by the PCR are once cloned using Escherichia coli or the like as a host, DNAs which are cloned respectively into plural clones are prepared, and nucleotide sequences of the DNAs may be analyzed. By obtaining a ratio of samples in which a base detected at a position corresponding to cytosine to be analyzed is cytosine among samples to be analyzed, a methylation frequency of cytosine to be analyzed may be also measured.

As a third method, a DNA is first extracted from a mammal-derived specimen, for example, using a commercially available DNA extracting kit or the like.

Incidentally, when blood is used as a specimen, plasma or serum is prepared from blood according to the conventional method, and a free DNA (including a DNA derived from cancer cells such as breast cancer cells) contained in the prepared plasma or serum as a specimen is analyzed, whereby, a DNA derived from cancer cells such as breast cancer cells can be analyzed while avoiding a hemocyte-derived DNA, a sensitivity for detecting cancer cells such as breast cancer cells, a tissue containing it and the like can be improved.

There is also a method in which, then, after the extracted DNA is contacted with a reagent for modifying unmethylated cytosine, a DNA containing cytosine in one or more nucleotide sequence (s) represented by CpG present in a nucleotide sequence of a promoter region or a coding region of 3OST gene, and a probe which can distinguish the presence or the absence of methylation of cytosine to be analyzed are hybridized and, thus, the presence or the absence of binding of the DNA with the probe is investigated.

Herein, for example, when 3OST gene is 3OST2 gene, examples of one or more nucleotide sequence (s) represented by CpG present in a nucleotide sequence of a promoter region or a coding region of 3OST gene include a nucleotide sequence of a genomic DNA containing an exon 1 of human-derived 3OST2 gene and a promoter region situated on a 5' upstream side thereof, more specifically, the nucleotide sequence represented by SEQ ID NO: 1 (corresponding to the nucleotide sequence represented by nucleotide numbers 57001 to 58999 of the nucleotide sequence described in Genbank Accession No. HUAC003661). In the nucleotide sequence represented by SEQ ID NO: 1, the ATG codon encoding methionine at the amino-terminal of human-derived 3OST2 protein is represented by nucleotide numbers 1514 to 1516, and the nucleotide sequence of the exon 1 is representedby nucleotide numbers 1514 to 18999. Cytosine in a nucleotide sequence represented by CpG present in the nucleotide sequence represented by SEQ ID NO: 1, inter alia, cytosine in CpG present in a region in which CpGs are densely present in the nucleotide sequence represented by SEQ ID NO: 1 shows a high methylation frequency (i.e. hypermethylation status) in cancer cells such as breast cancer cells. More specifically, examples of cytosine having a high methylation frequency in a breast cancer cell include cytosines represented by nucleotide numbers 1239, 1243, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence represented by SEQ ID NO: 1.

It is better to design a probe used in the hybridization based on a nucleotide sequence containing cytosine to be analyzed in view of that cytosine which has not undergone methylation is converted into uracil, and cytosine which has undergone methylation is not converted into uracil. That is, a methylation-specific probe having a nucleotide sequence when methylated cytosine is contained [a nucleotide sequence in which cytosine at a position to be methylated (cytosine in CpG) still remains as cytosine, and unmethylated cytosine (cytosine not contained in CpG) is converted into uracil] or a nucleotide sequence complementary to such the nucleotide sequence, and a unmethylation-specific probe having a nucleotide sequence when cytosine is not methylated (a nucleotide sequence in which all cytosines are converted into uracil) or a nucleotide sequence complementary to such the nucleotide sequence are designed. Such the probe may be used after labeled, in order to facilitate analysis of the presence or the absence of binding of a DNA with the probe. Alternatively, a probe may be used by immobilizing on a carrier according to the conventional method. In this case, it is better to pre-label a DNA extracted from a mammal-derived specimen.

As a reagent for modifying unmethylated cytosine, bisulfite such as sodium bisulfite and the like can be used.

In order that the extracted DNA is contacted with a reagent for modifying unmethylated cytosine, for example, the DNA is first treated with bisulfite such as sodium bisulfite and the like (concentration in a solution: for example, final concentration 3M) at 55°C for around about 10 to 16 hours (overnight) in an alkaline solution (pH 9 to 14). In this case, unmethylated cytosine is converted into uracil and, on the other hand, methylated cytosine is not converted into uracil, and still remains as cytosine.

If necessary, by performing PCR using a DNA treated with bisulfite or the like as a template in the same manner as the second method, the DNA may be amplified in advance.

Then, a DNA treated with bisulfite or the like or the DNA pre-amplified by PCR is hybridized with a.probe which can distinguish the presence or the absence of methylation of cytosine to be analyzed. By comparing an amount of a DNA which binds with a methylation-specific probe, and an amount of a DNA which binds with an unmethylation-specific probe, a methylation frequency of cytosine to be analyzed can be measured.

More specifically, when 3OST gene is 3OST2 gene, a probe for measuring a methylation frequency of the gene can be designed as described above, for example, based on a nucleotide sequence containing one or more cytosine (s) in CpG present in a nucleotide sequence which is in a promoter region or a coding region of 3OST2 gene. For example, probes can be designed based on a nucleotide sequence containing one or more cytosine (s) in CpG present in a region in which CpGs are densely present in the nucleotide sequence represented by SEQ ID NO: 1, specifically, cytocine (s) represented by nucleotide numbers 1239, 1243, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence represented by SEQ ID NO: 1. Examples of such the probe are shown below.

### <Set 1>

Unmethylation-specific prove: 5'-TGTTTTGAATTTGGTGTAT-3' (SEQ
ID NO: 14)

Methylation-specific prove: 5'-CGTTTCGAATTCGGCGTAC-3' (SEQ ID
NO: 15)

### <Set 2>

Unmethylation-specific prove: 5′-TTGGGATTTTTGGTATTGTG-3′
(SEQ ID NO: 16)

Methylation-specific prove: 5'-TCGGGATTTTTGGTATTGTG-3' (SEQ
ID NO: 17)

Hybridization can be performed according to the conventional method, for example, described in Sambrook J., Frisch E. F., Maniatis T., Molecular Cloning 2^{nd} edition, published by Cold Spring Harbor Laboratory press, and the like. Hybridization is usually performed under the stringent conditions. Herein, examples of the "stringent conditions" include the conditions under which a hybrid is formed at 45°C in a solution containing 6 × SSC (a solution containing 1.5M NaCl and 0.15M trisodium citrate is 10 x SSC) and, thereafter, the hybrid is washed with 2 × SSC at 50°C (Molecular Biology, John Wiley and Sons, N.Y.(1989), 6.3.1-6.3.6), and the like. The salt concentration in the washing step can be selected, for example, from the conditions of 2 × SSC and 50°C (low stringency condition) to the conditions of 0.2 × SSC and 50°C (high stringency conditions). A temperature in a washing step can be selected, for example, from room temperature (low stringency condition) to 65°C (high stringency condition). Alternatively, both of the salt concentration and temperature may be changed.

After such the hybridization is performed, a methylation frequency of cytosine to be analyzed (i.g. cytosine in CpG contained in a nucleotide sequence which is a basis for probe design) can be measured by comparing an amount of a DNA which binded with a methylation-specific probe, and an amount of a DNA which binded with an unmethylation-specific probe.

As a fourth method, a DNA is first extracted from a mammal-derived specimen, for example, using a commercially available DNA extracting kit or the like.

Incidentally, when blood is used as a specimen, plasma or serum is prepared from blood according to the conventional method, and a free DNA (including a DNA derived from cancer cells such as breast cancer cells) contained in the prepared plasma or serum as a specimen is analyzed, whereby, a DNA derived from cancer cells such as breast cancer cells can be analyzed while avoiding a hemocyte-derived DNA, and a sensitivity for detecting cancer cells such as breast cancer cells, a tissue containing it, or the like can be improved.

There is also a method in which, then, the extracted DNA is reacted with a restriction enzyme which can distinguish the presence or the absence of methylation of cytosine to be analyzed and, thereafter, the presence or the absence of digestion with the restriction enzyme is investigated. Herein, when 3OST gene is 3OST2 gene, examples of one or more nucleotide sequence(s) represented by CpG present in a nucleotide sequence of a promoter region or a coding region of 3OST gene include a nucleotide sequence of a genomic DNA containing an exon 1 of human-derived 3OST2 gene and a promoter region situated on a 5' upstream side thereof, more specifically, the nucleotide sequence represented by SEQ ID NO: 1 (corresponding to the nucleotide sequence represented by nucleotide numbers 57001 to 58999 in the nucleotide sequence described in Genbank Accession No. HUAC003661). In the nucleotide sequence represented by SEQ ID NO: 1, the ATG codon encoding methionine at the amino-terminal of human-derived 3OST2 protein is represented by nucleotide numbers 1514 to 1516, and the nucleotide sequence of the exon 1 is represented by nucleotide numbers 1514 to 18999. Cytosine in a nucleotide sequence represented by CpG present in the nucleotide sequence represented by SEQ ID NO: 1, inter alia, cytosine in CpG present in a region in which CpGs are densely present in the nucleotide sequence represented by SEQ ID NO: 1 shows a high methylation frequency (i.e. hypermethylation status) , for example, in cancer cells such as breast cancer cells. More specifically, examples of cytosine having a high methylation frequency in a breast cancer cell include cytosines represented by nucleotide numbers 1239, 1243, 1248, 1252, 1260, 1271, 1281, 1303, 1314, 1322, 1331, 1351, 1372, 1381, 1384, 1393, 1397, 1402, 1404, 1410, 1418, 1423, 1425 and the like in the nucleotide sequence represented by SEQ ID NO: 1.

The "restriction enzyme which can distinguish the presence or the absence of methylation of cytosine" (hereinafter, referred to as methylation-sensitive restriction enzyme in some cases) used in the method means a restriction enzyme which does not digest a recognition sequence containing methylated cytosine, and can digest a recognition sequence containing unmethylated cytosine. In the case of a DNA in which cytosine contained in a recognition sequence is methylated, the DNA is not cut even when a methylation-sensitive restriction enzyme is acted thereon and, on the other hand, in the case of a DNA in which cytosine contained in a recognition sequence is not methylated, the DNA is cut when a methylation-sensitive restriction enzyme is acted thereon. Examples of the methylation-sensitive enzyme include HpaII, BstUI and the like.

Examples of a method for investigating the presence or the absence of digestion with the restriction enzyme include a method for investigating the presence or the absence of amplification of a DNA (amplification product) by performing PCR using the DNA as a template and using a primer pair which can amplify a DNA containing cytosine to be analyzed in a recognition sequence and not containing a recognition sequence for the restriction enzyme in addition to that recognition sequence. When cytosine to be analyzed is methylated, an amplification product is obtained. On the other hand, when cytosine to be analyzed is not methylated, an amplification product is not obtained. Like this, by comparing with an amount of the amplified DNA, a methylation frequency of cytosine to be analyzed can be measured.

For example, in the case of cytosine represented by the nucleotide number 1216 in the nucleotide sequence represented by SEQ ID NO: 1, the cytosine is contained in a recognition sequence for HpaII, and a methylation frequency of the cytosine can be measured by the aforementioned method.

Further, examples of other method for investigating the presence or the absence of digestion of the restriction enzyme include a method in which Southern hybridization is performed on a DNA which contains cytosine to be analyzed in a recognition sequence and has been reacted with a methylation-sensitive restriction enzyme, using, as a probe, a DNA which is derived from 3OST gene such as 3OST2 gene and does not contain a recognition sequence for the restriction enzyme, and a length of the hybridized DNA is investigated. When cytosine to be analyzed is methylated, a longer DNA is detected, as compared with the case where the cytosine is not methylated. By comparing an amount of the detected longer DNA and an amount of the shorter DNA, a methylation frequency of cytosine to be analyzed can be measured.

Using the aforementioned various methods, a methylation frequency of 3OST gene contained in a mammal-derived specimen is measured. By comparing the measured methylation frequency, and for example a methylation frequency (control) of 3OST gene contained in a healthy mammal-derived specimen which can be diagnosed not to have cancer cells such as breast cancer cells, a cancerous state of the specimen is determined based on a difference obtained by the comparison. If a methylation frequency of 3OST gene contained in a mammal-derived specimen is higher as compared with a control (if 3OST gene is in a hypermethylation status as compared with a control) , it can be determined that a cancerous state of the specimen is higher as compared with a control.

Herein, the "cancerous state" has the same meaning as that generally used in the art, specifically, for example, the cancerous state means a malignancy of the cell when a mammal-derived specimen is a cell, and means an amount of cancer cells existing in the tissue when a mammal-derived specimen is a tissue.

When 3OST gene is 3OST2 gene, expression of the gene is lower in cancer cells such as breast cancer cells than in a specimen such as a cell and a tissue derived from a healthy mammal. Since a methylation frequency of the gene is higher in cancer cells such as breast cancer cells, the gene can not be normally expressed and, as a result, an amount of an expression product of the gene (more specifically, an amount of a transcription product or an amount of a translation product) is decreased. Like this, the present assessing method and the like, in place of a methylation frequency, an index value having the correlation therewith (in the above case, the value is an amount of an expression product and an index value having the negative correlation) may be measured.

That is, in the present assessing method, a cancerous state of a specimen can be determined based on a difference obtained by measuring an index value (e.g. an amount of an expression product) having the correlation with a methylation frequency of 3OST gene such as 3OST2 gene contained in a mammal-derived specimen, and comparing the measured index value (e.g. an amount of an expression product) having the correlation with the aforementioned methylation frequency with a control.

Examples of a method for measuring an index value having the correlation with an methylation frequency of 3OST gene such as 3OST2 gene contained in a mammal-derived specimen in the first step of the present assessing method include a method for measuring an amount of a mRNA which is a transcription product of 3OST gene such as 3OST2 gene. For the measurement, the known methods such as a RT-PCR method, a Northern blotting method [Molecular cloning, Cold Spring Harbor Laboratory (1989)], an in situ RT-PCR method [Nucleic acids Res., 21, 3159, 3166(1993)], an in situ hybridization method, and a NASBA method [Nucleic acid sequence-based amplification, nature, 350, 91-92 (1991)] may be used.

A sample containing a mRNA which is a transcription product of 3OST gene such as 3OST2 gene which is contained in a mammal-derived specimen may be prepared from the specimen by extraction, purification or the like according to the conventional method.

When the Northern blotting method is used for measuring an amount of a mRNA contained in the prepared sample, it is enough that a detecting probe contains 3OST gene or a part thereof (around about 100 bp to about 1000 bp oligonucleotides obtained by cutting 3OST gene such as 3OST2 gene with a restriction enzyme, or chemically synthesizing them according to a nucleotide sequence of 3OST gene such as 3OST2 gene) , and is not particularly limited as far as it imparts specificity which can be detected under the detecting conditions used in hybridization with a mRNA contained in the sample.

When the RT-PCR method is used for measuring an amount of a mRNA contained in the prepared sample, it is enough that a primer used can specifically amplify only 3OST gene such as 3OST2 gene, and a region tobe amplified and a number of nucleotides are not particularly limited. Examples of such the primer include primers (S: sense, A: antisense) and the like shown below. Using these primers, an amount of a transcription product may be also measured by the RT-PCR method as shown in Examples later.

### <Case where 3OST gene is 3OST2 gene>

S: 5'-CGTGCTGGAGTTTATCCGAGT-3' (SEQ ID NO: 18)

A; 5'-CAAAGTAGCTGGCGTCTTCT-3' (SEQ ID NO: 19)

Examples of other method for measuring an index value having the correlation with a methylation frequency of 3OST gene such as 3OST2 gene which is contained in a mammal-derived specimen in the first step of the present assessing method include a method for measuring an amount of an OST2 protein such as a 3OST2 protein which is a translation product of 3OST gene such as 3OST2 gene. For the measurement, the known methods such as an immunoblotting method, a separating method by immunoprecipitation, and an indirect competitive inhibiting method (ELISA method) described in Cell Technology Handbook, Yodosha, 207 (1992), and the like, using a specific antibody (monoclonal antibody, polyclonal antibody) against a OST2 protein such as a 3OST2 protein may be used.
Incidentally, a specific antibody against an OST2 protein such as a 3OST2 protein can be prepared according to the conventional immunological method using the protein as an immune antigen.

Using the aforementioned various methods, an index value having the correlation with a methylation frequency of 3OST gene contained in a mammal-derived specimen is measured. By comparing an index value having the correlation with the measured methylation frequency, for example with an index value (control) having the correlation with the methylation frequency of the 3OST2 gene contained in a healthy mammal-derived specimen which can be diagnosed not to have cancer cells such as breast cancer cells, based on a difference obtained by the comparison, a cancerous state of the specimen is determined. If an index value having the positive correlation with a methylation frequency of 3OST gene contained in a mammal-derived specimen is higher as compared with a control, or if an index value having the negative correlation therewith is lower as compared with the control (if 3OST gene is in a hypermethylation status as compared with a control), it can be determined that a cancerous state of the specimen is higher as compared with a control.

A primer, a probe and a specific antibody which can be used in various methods for measuring a methylation frequency of 3OST gene such as 3OST2 gene or an index value having the correlation therewith in the present assessing method are useful as a reagent of a kit for detecting cancer cells such as breast cancer cells. The present invention also provides a kit for detecting cancer cells such as breast cancer cells which contains these primer, probe or specific antibody as a reagent, and a chip for detecting cancer cells such as breast cancer cells which comprises the primer, the probe, the specific antibody or the like immobilized on a carrier, and the right scope of the present assessing method of course includes use in a form of the aforementioned detecting kit and detecting chip utilizing substantial principle of the method.

When 3OST gene is 3OST2 gene, expression of the gene is lower in cancer cells such as breast cancer cells than in a specimen such as a cell and a tissue derived from a healthy mammal. On the other hand, as also shown in Examples later, by acting a substance inhibiting DNA methylation relating to 3OST gene such as 3OST2 gene on a cancer cell such as a breast caner cell and the like, an amount of an expression product of the gene can be increased. This means that a substance which can compensate reduction in an expression level of 3OST gene such as 3OST2 gene in cancer cells such as breast cancer cells or function reduction accompanied therewith-for example, 3OST gene such as 3OST2 gene in which methylation abnormality as recognized in cancer cells such as breast cancer cells has not occurred [J. Biol. Chem., 274, 5170-5184(1999)], an expression product of the gene, a substance having the ability of promoting the expression of 3OST gene such as 3OST2 gene (e.g. substance which inhibits DNA methylation relating to 3OST gene such as 3OST2 gene, a substance which reduces a methylation frequency of 3OST gene such as 3OST2 gene) and the like are useful in treating a cancer such as a breast cancer and the like, and inhibiting canceration of a normal tissue such as breast tissue, mammary gland tissue and mammary gland epithelial tissue.

For example, canceration would be inhibited by administering a substance which reduces a methylation frequency of 3OST gene such as 3OST2 gene to cells in a body of a mammal which can be diagnosed to be cancer. Further, for example, cancer cells such as breast cancer cells is provided with a substance which inhibits DNA methylation relating to 3OST gene such as 3OST2 gene, whereby, cytosine in CpG present in a nucleotide sequence in a promoter region or a coding region of 3OST gene such as 3OST2 gene would be in a hypomethylation status like a normal tissue, an expression amount of a mRNA which is a transcription product of 3OST gene such as 3OST2 gene would be increase and, consequently, an expression amount of a 3OST protein such as a 3OST2 protein and the like which is a translation product of 3OST gene such as 3OST2 gene could be increased. Further, for example, by introducing into cancer cells such as breast cancer cells 3OST gene such as 3OST2 gene or a cDNA comprising a nucleotide sequence encoding an amino acid sequence of a 3OST protein such as a 3OST2 protein and the like, an expression amount of a 3OST protein such as 3OST2 protein and the like in cancer cells such as breast cancer cells could be increased.

That is, the present invention also provides (1) an anti-cancer agent, which comprises a substance having the ability of promoting the expression of 3OST gene such as 3OST2 gene as an active ingredient, wherein the active ingredient is formulated into a pharmaceutically acceptable carrier, and (2) an anti-cancer agent, which comprises a nucleic acid consisting of a nucleotide sequence encoding an amino acid sequence of 3OST gene such as a 3OST3 gene as an active ingredient, wherein the active ingredient is formulated into a pharmaceutically acceptable carrier (hereinafter, collectively referred to as the present anti-cancer agent in some cases).

A dosage form of the present anti-cancer agent is not particularly limited as far as it is a conventional preparation, and such the preparation can be prepared, for example by incorporating an active ingredient into a pharmaceutically acceptable carrier such as a water-soluble solvent, a non-water-soluble solvent, a buffer, a solubilizer, an isotonic agent, and a stabilizer. If necessary, a supplementing agent such as an antiseptic, a suspending agent, and an emulsifying agentmay be added. In addition, whenadministeredparenterally (generally, preferably by an injection and the like), the anti-cancer agent can be used in the form of a conventional liquid preparation such as a solution and the like.

An effective amount of the present anti-cancer agent can be administered parenterally to mammals such as a human being (e.g. a cell in a body of a mammal which can be diagnosed to be cancer). Examples of a method for parenterally administering the agent include an injection (subcutaneously, intravenously, and locally) and the like.

A dose is different depending on age, sex and weight of a mammal to be administered, a degree of disease, a kind and an administration form of the present anti-cancer agent and the like and, usually, an active ingredient may be administered at an amount resulting in an intracellular level equivalent to such the concentration level that an active ingredient works effectively in a patient cell. Furthermore, the aforementioned dose per day can be administered once or by dividing into a few times.

Herein, examples of a method for introducing 3OST gene such as 3OST2 gene into a cell include a gene introducing method utilizing a virus vector, a gene introducing method utilizing a non-virus vector (Nikkei Science, 1994, April, p 20-45, Experimental Medicine, Extra Edition, 12(15) (1994), Experimental Medicine Separate Volume "Fundamental Technique of Gene Therapy", Yodosha (1996)) and the like.

Examples of the former gene introducing method include a method for introducing the gene by incorporating a DNA encoding TR4 or mutant TR4 into DNA virus or RNA virus such as retrovirus, adenovirus, adeno-associatedvirus, herpesvirus, vacciniavirus, poxvirus, poliovirus, cinbisvirus and the like. In addition, examples of the gene introducing method utilizing a non-virus vector include a method for administering an expression plasmid directly into muscle (DNA vaccine method), a liposome method, a lipofectine method, a microinjection method, a calcium phosphate method, an electroporation method and the like.

In addition, examples of a method utilizing a DNA of a 3SOT gene such as a 3SOT2 gene as an active ingredient of a gene therapeutic as an anti-cancer agent include an in vivo method for introducing a DNA of the gene directly into a body, an ex vivo method for taking out a particular cell of a human, introducing a DNA of the gene into the cell outside a body, and returning the cell into a body (Nikkei Science, 1994, April, p 20-45, Monthly Pharmaceutical Affairs, 36(1), 23-48 (1994), Experimental Medicine, Extra Edition, 12(15)(1994)) and the like.

In the case of the former in vivo method, a DNA of the gene can be administered via a suitable administration route depending on disease, symptom and the like. For example, the DNA can be administered to mammary gland tissue or a breast cancer cell, or intravenously, intraarterially, subcutaneously, intradermally or intramuscularly by an injection.

A dosage form of the gene therapeutic agent as an anti-cancer agent may be a suspension, or a liposome preparation such as a frozen agent, a centrifugation concentration frozen agent and the like in addition to an injectable. Such the preparation can be prepared by incorporating the gene (including a form of the gene of a vector type or a virus type, or a plasmid type) into a pharmaceutically acceptable carrier such as a water-soluble solvent, a non-water-soluble solvent, a buffer, a solubilizer, an isotonic agent, anda stabilizer. If necessary, a supplementing agent such as an antiseptic, a suspending agent, an emulsifying agent may be added. In addition, when parenterally administered (generally, preferably by an injection or the like), the anti-cancer agent can be used in the form of a conventional liquid preparation such as a solution and the like.

The present searching method is a method for searching a substance having the ability of promoting the expression of 3OST gene such as 3OST2 gene, and has (1) a first step of bringing a test substance into contact with a cancer cell, (2) a second step of measuring an amount of an expression product of 3OST gene contained in the cancer cell after the first step (1), and (3) a third step of determining the ability of promoting the expression of 3OST gene possessed by the test substance based on a difference obtained by comparing the measued amount of an expression product with a control.

A cancer cell in the first step of the present searching method is not particularly limited, and may be a cancer cell separated from a mammal-derived cancer tissue, or a mammal-derived cancer cell line which is established as a cell line. Examples of the mammal include human being, monkey, mouse, rat, hamster and the like. Preferable examples of the cancer include a breast cancer and the like. Specifically, embodiments thereof include the known human-derived breast cancer cell line such as MCF-7 (available fromATCC), ZR75-1 (available fromATCC) , SK-BR3 (available from ATCC), YMB-lE(available JCRB), T-47D (available from ATCC), MDA-MB-231 (available from ATCC), and MDA-MB-468 (available from ATCC).

An amount of a cancer cell for bringing a test substance into contact with a cancer cell in the first step of the present searching method is usually about 10⁴ to 10⁸ cells, preferably about 10⁵ to 10⁷ cells. The concentration of a test substance is usually about 0.1ng/ml to about 100 µg/ml, preferably about 1ng/ml to about 50 µg/ml. A time period for bringing a test substance into contact with a cancer cell is usually 1 hour to around 5 days, preferably a few hours to around 2 days. A number of times for bringing a test substance into contact with a cancer cell may be once or plural times.

The environment under which a test substance is contacted with a cancer cell is preferably the environment under which vital activity of a cancer cell is maintained, for example, the environment under which the energy source of the cancer cell coexists. Specifically, it is advantageous that the first step is performed in a medium.

For measuring an amount of an expression product of 3OST gene contained in a cancer cell in the second step of the present searching method, the amount may be measured according to the aforementioned "method for measuring an index value having the correlation with a methylation frequency of 3OST gene such as 3OST2 gene contained in a mammal-derived specimen in the first step of the present assessing method" and the like.

For determining the ability of promoting the expression of 3OST gene possessed by a test substance based on a difference obtainedby comparing an amount of an expression product measured in the second step of the present searching method with a control, as described above, the measured amount of an expression product is compared, for example, with an amount (control) of an expression product of 3OST gene when the concentration of a test substance for bringing a test substance into contact with a cancer cell in the first step of the present searching method is zero (that is, when a test substance is not contacted with a cancer cell), whereby, the ability of promoting the expression of 3OST gene such as 3OST2 gene possessed by a test substance is determined based on a difference obtained by the comparison. If an amount of an expression product of 3OST gene such as 3OST2 gene contained in a cancer cell which has been contacted with a test substance is higher when compared with a control (in this case, an amount of an expression product of 3OST gene such as 3OST2 gene contained in a cancer cell which has not been contactedwith a test substance), it can be determined that the test substance has the ability of promoting the expression of 3OST gene such as 3OST2 gene. Of course, as a control, an amount of an expression product of 3OST gene such as 3OST2 gene when other test substance is contacted with a cancer cell may be used and, in this case, it is preferable that the ability of promoting expression for 3OST gene such as a 3SOT2 gene possessed by the other test substance is known in advance.

Like this, it is possible to search a substance having the ability of promoting the expression of 3OST gene such as 3OST2 gene. In addition, it is preferable that an amount of an expression product of 3OST gene such as 3OST2 gene contained. in a specimen derived from a normal cell line such as a normal mammary gland epithelial cell line, or a healthy mammal which can be diagnosed not to have cancer cells such as breast cancer cells is measured as a background or a control in both of the case where a test substance is contacted and the case where a test substance is not contacted.

### EXAMPLES

The present invention will be explained in detail below by way of Examples, but the present invention is not limited by them.

### Example 1 (Test of confirming methylation status of 3OST2 gene in breast cancer cell line (1))

Seven kinds of human-derived breast cancer cell lines [MCF-7 (ATCC), ZR-75-1 (ATCC), SK-BR3(ATCC), YMB-1 (JCRB), T-47D(ATCC), MDA-MB-231(ATCC), MDA-MB-468 (ATCC)] and human-derived normal mammary gland epithelial cell line [HMEC (Clonetics) ] were cultured to confluent in a medium exclusively used for each cell line described in catalogs of ATCC (American Type Culture Collection) , JCRB (Japanese Cancer Research Bank) and Clonetics, and thereafter about 2 × 10⁷ cells were collected, respectively. 10-Fold volume of a SEDTA buffer [10 mM Tris/HCl (pH 8.0), 10 mM EDTA (pH 8.0), 100 mM NaCl] was added to the collected cells, and this was homogenized. To the resulting mixture were added proteinase K (Sigma) of 200 µg/ml and sodium dodecylsulfate of the amount to give a concentration of 1% (w/v) , and this was shaken at 55°C for about 16 hours. After completion of shaking, the mixture was treated by phenol [saturated with 1M Tris/HCl (pH 8.0)]-chloroform extraction. The aqueous layer was recovered, and NaCl was added thereto to give a concentration of 0.5N, and this was ethanol-precipitated to recover the precipitates. The recovered precipitates were dissolved in a TE buffer (10 mM Tris, 1 mM EDTA, pH 8.0), and RNase A (Sigma) was added thereto to give a concentration of 40 µg/ml, followed by incubation at 37°C for 1 hour. The incubated mixture was treated by phenol-chloroform extraction. The aqueous layer was recovered, NaCl was added thereto to give a concentration of 0.5N, and this was ethanol-precipitated to recover precipitates (genomic DNA). The recovered precipitates were rinsed with 70% ethanol to obtain a genomic DNA.

The resulting genomic DNAwas treated with sodiumbisulfite according to the method described in Clark et al., Nucl. Acids. Res., 22, 2990-2997, 1994; Herman et al., Pro. Natl. Acad. Sci. USA, 93, 9821-9826, 1996. That is, the aforementioned genomic DNA (0.2-1 µg) was dissolved in a TE buffer to prepare 20 µl of a genomic DNA solution, about 2 µl of 6M sodium hydroxide was added thereto and, thereafter, the mixture was allowed to stand at room temperature for 15 minutes. To the stood mixture were added 9 µl of 10 mM hydroquinone (Sigma) and 120 µl of 3.6N sodiumbisulfite (Sigma) , and this was incubated at 55°C overnight. A DNA was purified from the incubated solution using Wizard DNA clean up system (Promega). The purified DNA was dissolved in 50 µl of a TE buffer, and 5 µl of 6M sodium hydroxide was added thereto and, thereafter, the mixture was allowed to stand at room temperature for 5 minutes. Then, the stood mixture was ethanol-precipitated to recover precipitates (DNA). The recovered precipitates were suspended in 50 µl of a TE buffer.

In order to amplify a DNA having a nucleotide sequence represented by nucleotide numbers 1218 to 1376 in the nucleotide sequence represented by SEQ ID NO: 1 (DNA corresponding to nucleotide numbers 58218 to 58376 of a 3OST2 gene represented by Genbank Accession No. AC003661; 150 bp) by PCR using the resulting DNA as a template, primers containing of following nucleotide sequence were synthesized:

BU1: 5′ -GTGTATGTAAGAGTTTGGGAG-3′ (SEQ ID NO: 10)

BM1: 5'-GCGTACGTAAGAGTTTGGGAG-3' (SEQ ID NO: 11)

BU2: 5'-CTCCAAAAACTACTAACCAAAATAC-3' (SEQ ID NO: 12)

BM2: 5'-CTCCGAAAACTACTAACCAAAATAC-3' (SEQ ID NO: 13)

A reaction solution for PCR was used which was obtained by mixing 20 ng of a DNA as a template, each 1 µl of four kinds of 30 pmol/µl aforementioned primer solutions, 3 µl of 2 mM dNTP, 3 µl of 10 × buffer (100 mM Tris-HCl pH 8.3, 500 mM KCl, 20 mM MgCl₂) and 0.2 µl of a 5U/µl thermostable DNA polymerase, and adding sterilized ultrapure water thereto to an amount of 30 µl solution. The reaction solution was retained at 94°C for 10 minutes, and PCR was performed under the conditions of 40 cycles of temperature maintenance, each cycle being 30 seconds at 94°C, 60 seconds at 55°C and 45 seconds at 72°C. After PCR was performed, the reaction solution of PCR containing the amplification product was subjected to 2% agarose gel electrophoresis.

A part containing a 159 bp DNA was excised from the electrophoresed agarose gel, and a DNA was extracted. The extracted DNA was ligated with pGEM-T-Easy (Promega) , and this was introduced into a Escherichia coli XL1Blue strain. Transformed Escherichia coli colony was picked up at 10 per one breast cancer cell line, each was lysed. PCR was performed under the aforementioned conditions using the resulting Escherichia coli lysed solution as a template. The reaction solution of PCR was subjected to agarose gel electrophoresis as described above, a part containing a 159 bp DNA was excised, and a DNA was extracted. A nucleotide sequence of the extracted DNA was analyzed with a DNA sequencer (ABI310 type, PE Biosystems). The sequence results of the DNA prepared from each colony, whether cytosine in CpG present in a region represented by nucleotide numbers 1218 to 1376 in the nucleotide sequence represented by SEQ ID NO: 1 was replaced with uracil or not was investigated, and a frequency of DNAs in which cytosine is not replaced with uracil among DNAs prepared from tested 10 colonies, that is, a methylation frequency of the cytosine was measured.

The results are shown in Table 1. In the case of the DNA prepared from a normal mammary gland epithelial tissue-derived cell (HMEC) , a DNA in which cytosine was not replaced with uracil (that is, metylated DNA) was not found in 6 cytosines (nucleotide numbers 1239, 1243, 1248, 1252, 1260, 1271) in CpG present in a region represented by nucleotide numbers 1239 to 1271. On the other hand, in the case of DNAs prepared from seven kinds of breast cancer cell lines, it was found that they are DNAs in which almost of cytosines in CpG present in region were not replaced with uracil (i.e. methylated DNA).

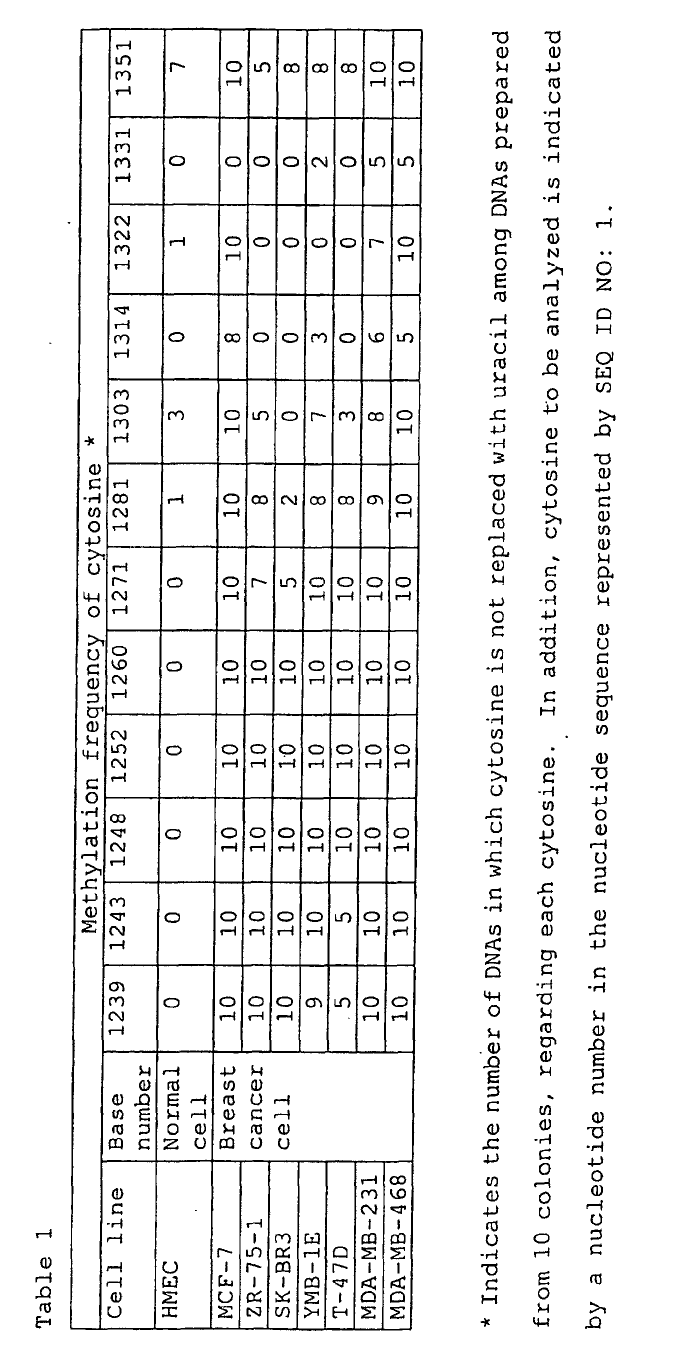

### Example 2 (Test of confirming expression status of 3OST2 gene in breast cancer cell line)

Seven kinds of human-derived breast cancer cell lines (MCF-7, ZR-75-1, SK-BR3, YMB-1E, T-47D, MDA-MB-231, MDA-MB-468) and human-derived normal mammary gland epithelial cell (HMEC) were cultured to 70% confluent in RPMI or DMEM or in an exclusively used medium, and thereafter, each cell was corrected. 1ml of an ISOGEN solution (Nippon Gene) was mixed with collected each cell (wet weight about 100mg) , this was homogenized, and 0.2ml of chloroform was added thereto to suspend them. After suspending, the mixture was centrifuged (4°C, 15000 × g, 15 minutes) to recover the supernatant. 0.5ml isopropanol was added to the recovered supernatant to suspend them, and the suspension was centrifuged (4°C, 15000 × g, 15 minutes) to recover the precipitates (RNA). The recovered precipitates were rinsed with 75% ethanol, and dissolved in DEPC (diethyl pyrocarbonate)-treated water.

The thus obtained RNA was treated with DNaseI (Life Technologies), and this was used as a template and Superscript II (Life Technologies) was used to synthesize a cDNA according to a protocol attached to the enzyme. By performing PCR using the synthesized cDNA as a template and using 3OST2 S and 3OST2 A shown below as a primer pair, a DNA derived from a mRNA of 3OST2 gene was amplified. Thereupon, as a control by performing PCR using the aforementioned cDNA as a template and using GAPDH S and GAPDH A solution below as a primer pair, a DNA derived from a mRNA of a GAPDH gene was amplified.

### <Primer ( S: sense, A: antisense)>

3OST2 S: 5′-CGTGCTGGAGTTTATCCGAGT-3′ (SEQ ID NO: 18)

3OST2 A: 5'-CAAAGTAGCTGGGCGTCTTCT-3' (SEQ ID NO: 19)

GAPDH S: 5′-AGGTGAAGGTCGGAGTCAACG-3′ (SEQ ID NO: 20)

GAPDH A: 5′-AGGGGTCATTGATGGCAACA-3′ (SEQ ID NO: 21)

A reaction solution PCR was used which was obtained by mixing 20 ng of a cDNA as a template, each 0.25 µl of two kinds of 25 pmol/µl aforementioned primer solution, 0.5 µl of 10 mM dNTP, 2.5 µl of 10 × buffer (100 mM Tris-HCl pH 8.3, 500 mM KCl, 20 mM MgCl₂) and 0. 15 µl of a 5U/µl thermostable DNA polymerase, and adding sterilized ultrapure water thereto to an amount of solution of 25 µl. When a DNA derived from mRNA of 3OST2 gene was amplified, PCR was performed at the conditions under which the reaction solution was retained at 94°C for 10 minutes, 28 cycles of temperature maintenance were performed, each cycle was 30 seconds at 94°C, 30 seconds at 45°C and 60 seconds at 72°C, and a temperature was maintained at 72°C for 10 minutes. In addition, when a DNA derived from a mRNA of a GAPDH gene was amplified, PCR was performed at the conditions under which the reaction solution was retained at 95°C for 10 minutes, 18 cycles of temperature maintenance were performed, each cycle was 30 seconds at 94°C, 30 seconds at 55°C and 60 seconds at 72°C, and a temperature was retained at 72°C for 10 minutes. In any case, after PCR was performed, the PCR reaction solution containing the amplification product was subjected to 2% agarose gel electrophoresis.

The results are shown in Figure 1. In the case of human-derived normal mammary gland epithelial cell (HMEC), a DNA (161 bp) derived from a mRNA of 3OST2 gene was detected, while in any case of seven kinds of breast cancer cell lines, the DNA was not detected. A DNA derived from a mRNA of a GAPDH gene was detected similarly in any case of a normal mammary gland epithelial cell (HMEC) and seven kinds of breast cancer cell lines. That is, in human-derived normal mammary gland epithelial cell (HMEC) , expression of 3OST2 gene was confirmed, while in any of seven breast cancer cell lines, expression of 3OST2 gene was not recognized.

### Example 3 (Test confirming methylation status of 3OST2 gene in breast cancer cell line (2) and effect of methylation inhibitor on expression of the gene)

Two kinds of human-derived breast cancer cell lines (MCF-7, MDA-MB-468) and human-derived normal mammary gland epithelial cell (HMEC) were seeded at a density of 3 to 6 × 10⁵ cells/10cm plate, and cultured using RPMI or DMEM or an exclusively used medium. On the first day after seeding, 5-aza-2'-deoxytidine (manufactured by Sigma) (hereinafter, referred to as 5Aza-dC) which is a methylation inhibitor was added to a medium to give the concentration of 0.5 to 6 µM. Twenty four hours after addition of 5Aza-Dc, the medium was exchanged with the aforementioned medium to which no 5Aza-dC was added, and culturing was continued. Then, on the third day after seeding, 5Aza-dC was added to the medium similarly. On the fourth day after seeding, cells were recovered, and a genomic DNA was extracted and recovered from the recovered cells according to the same manner as that of Example 1. Further, according to the same manner as that of Example 2, a RNA was extracted and recovered.

The extracted and recovered genomic DNA was treated with sodium bisulfite according to the same manner as that of Example 1. The resulting DNA was used as a template and PCR was performed using unmethylation-specific primers U2 and U3, or methylation-specific primers M3 and M4 shown below. When unmethylation-specific primers U2 and U3 are used, a 173 bp DNA corresponding to nucleotide numbers 1260 to 1432 in the nucleotide sequence represented by SEQ ID NO: 1 is amplified, while when methylation-specific primers M3 and M4 are used, a 172 bp DNA corresponding to nucleotide numbers 1252 to 1423 represented by SEQ ID NO: 1 is amplified.

### <Unmethylation-specific primers>

U2: 5′-AAAACTCACATAACACTACCACA-3′ (SEQ ID NO: 3)

U3: 5'-TGGAGTTTTATTGTTTAGGATT-3' (SEQ ID NO: 4)

### <Methylation-specific primers>

M3: 5'-CGGTTGTTCGGAGTTTTATC-3' (SEQ ID NO: 8)

M4: 5'-GTAACGCTACCACGACCACG-3' (SEQ ID NO: 9)

A reaction solution for PCR was used which was obtained by mixing 20 ng of a DNA as a template, each 1 µl of 30 pmol/µl aforementioned primer solutions, 3 µl of 2 mM dNTP, 3 µl of 10 × buffer (100 mL Tris-HCl pH 8.3, 500 mM KCl, 20 mM MgCl₂) and 0.2 µl of a 5U/µl thermostable DNA polymerase, and adding sterilized ultrapure water thereto to an amount of solution of 30 µl. When the aforementioned unmethylation-specific primer was used, PCR was performed at the conditions under which the reaction solution was retained at 94°C for 10 minutes, and 40 cycles of temperature maintenance were performed, each cycle was 30 seconds at 94°C, 60 seconds at 64°C and 45 seconds at 72°C. In addition, when the aforementioned methylation-specific primer was used, PCR was performed at the conditions under which the reaction solution was retained at 94°C for 10 minutes, and 40 cycles of temperature maintenance were performed, each cycle was 30 seconds at 94°C, 60 seconds at 64°C and 45 seconds at 72°C. In any case, after PCRwas performed, a reaction solution of PCR containing the amplification product was subjected to 2% agarose gel electrophoresis.

Results are shown in Figure 2. In the case of human-derived normal mammary gland epithelial cell (HMEC), when an unmethylation-specific primer was used (lane U) , a band of the amplified DNA was recognized, and when a methylation-specific primer was used (lane M), a band of the amplified DNA was not detected. Therefore, in the case of human-derived normal mammary gland epithelial cell (HMEC), it was determined that at least cytosines represented by nucleotide numbers 1260, 1271, 1281, 1410, 1418, 1423 and 1425, respectively, of the nucleotide sequence represented by SEQ ID NO: 1 were not methylated. In addition, since a DNAwas not amplified by a methylation-specific primer, it was determined that cytosine represented by a nucleotide number 1252 or 1404 was not also methylated. In addition, in the case of two kinds of breast cancer cell lines (MAD-MB-468, MCF-7) cultured in the absence of a methylation inhibitor (5Aza-dC 0 µM), when an unmethylation-specific primer was used (lane U) , a band of the amplified DNA was not detected, and when a methylation-specific primer was used (lane M) , a band of the amplified DNA was recognized. Therefore, under that condition, it was determined that cytosines represented by nucleotide numbers 1252, 1260, 1271, 1404, 1410, 1418 and 1423, respectively, of the nucleotide sequence represented by SEQ ID NO: 1 were methylated. In addition, since a DNAwas not amplified by an unmethylation-specific primer, it was determined that cytosine represented by nucleotide number 1281 or 1425 was also methylated.

On the other hand, in the case of a breast cancer cell line MAD-MB-468 cultured in the presence of 0.5 µM 5Aza-dC, when an unmethylation-specific primer was used (lane U), a band of the amplified DNA was clearly recognized, and when a methylation-specific primer was used (lane M), a band of a DNA was not detected. Therefore, under that condition, it was determined that cytosines represented by nucleotide numbers 1260, 1271, 1281, 1410, 1418, 1423 and 1425, respectively, of the nucleotide sequence represented by SEQ ID NO: 1 were not methylated in almost genomic DNAs.

In addition, also in the case of a breast cancer cell line MCF-7 cultured in the presence of 6 µM 5Aza-dC, when an unmethylation-specific primer was used (lane U), and when a methylation-specific primer was used (lane M), a band of the amplified DNA was detected at the same degree of the concentration. Therefore, under that condition, it was determined that cytosines represented by nucleotide numbers 1260, 1271, 1281, 1410, 1418, 1423 and 1425, respectively, of the nucleotide sequence represented by SEQ ID NO: 1 were not methylated in genomic DNAs of approximately half of cells.
From the foregoing results, it was made clear that the aforementioned methylation of cytosine in a breast cancer cell line is inhibited by a methylation inhibitor.

Then, the RNA extracted and recovered from MAD-MB-468 was treated with DNaseI (Life Technologies), and this was used as a template, and Superscript II (Life Technologies) was used to synthesize a cDNA according to a protocol attached to the enzyme. According to the same PCR as that of Example 2 except that a synthesized cDNA was used as a template, a DNA derived from a mRNA of 3OST2 gene was amplified. Thereupon, as a control, a DNA derived from a mRNA of a GAPDH gene was amplified as in Example 2.

The results are shown in Figure 3. In the case of human-derived normal mammary gland epithelial cell (HMEC), a DNA (161 bp) derived from a mRNA of 3OST2 gene was detected. In addition, in the case of a breast cancer cell line (MAD-MB-468 cultured in the absence of a methylation inhibitor (5Aza-dC 0 µM), a DNA (161 bp) derived from a mRNA of 3OST2 gene was not detected. On the other hand, in the case of MAD-MB-468 cultured in the presence of 0.5 and 1 µM 5Aza-dC, a DNA (161 bp) derived from a mRNA of 3OST2 gene was detected. In any case of a normal mammary gland epithelial cell (HMEC) , a breast cancer cell line (MAD-MB-468) cultured in the absence of 5Aza-dC, and MAD-MB-468 cultured in the presence of 0.5 and 1 µM 5Aza-dC, a DNA derived from a mRNA of a GAPDH gene was detected similarly. That is, in the case of breast cancer cell line MAB-MB-468, expression of 3OST2 gene was recognized in the presence of a methylation inhibitor.

From the foregoing results, it was made clear that the aforementioned methylation in a breast cancer cell line is inhibited by a methylation inhibitor, and 3OST2 gene is expressed in the presence of methylation inhibitor.

### INDUSTRIAL APPLICABILITY

The present invention can provide a method for assessing cancerous state of a mammal-derived specimen.

### FREE TEXT IN SEQUENCE LISTING

SEQ ID NO: 2
   Designed oligonucleotide primer for PCR
SEQ ID NO: 3
   Designed oligonucleotide primer for PCR
SEQ ID NO: 4
   Designed oligonucleotide primer for PCR
SEQ ID NO: 5
   Designed oligonucleotide primer for PCR
SEQ ID NO: 6
   Designed oligonucleotide primer for PCR
SEQ ID NO: 7
   Designed oligonucleotide primer for PCR
SEQ ID NO: 8
   Designed oligonucleotide primer for PCR
SEQ ID NO: 9
   Designed oligonucleotide primer for PCR
SEQ ID NO: 10
   Designed oligonucleotide primer for PCR
SEQ ID NO: 11
   Designed oligonucleotide primer for PCR
SEQ ID NO: 12
   Designed oligonucleotide primer for PCR
SEQ ID NO: 13
   Designed oligonucleotide primer for PCR
SEQ ID NO: 14
   Designed oligonucleotide for probe
SEQ ID NO: 15
   Designed oligonucleotide for probe
SEQ ID NO: 16
   Designed oligonucleotide for probe
SEQ ID NO: 17
   Designed oligonucleotide for probe
SEQ ID NO: 18
   Designed oligonucleotide primer for PCR
SEQ ID NO: 19
   Designed oligonucleotide primer for PCR
SEQ ID NO: 20
   Designed oligonucleotide primer for PCR
SEQ ID NO: 21
   Designed oligonucleotide primer for PCR

## Claims

1. A method for assessing a cancerous state of a mammal-derived specimen, which comprises:
(1) a first step of measuring a methylation frequency of Heparan sulfate D-glucosaminyl 3-0-sulfotransferase gene contained in a mammal-derived specimen or an index value having the correlation therewith, and
(2) a second step of determining a cancerous state of the specimen based on a difference obtained by comparing the measured methylation frequency or the index value having the correlation therewith, with a control.

2. The assessing method according to claim 1, wherein Heparan sulfate D-glucosaminyl 3-0-sulfotransferase gene is Heparan sulfate D-glucosaminyl 3-0-sulfotransferase-2 gene.

3. The assessing method according to claim 1, wherein the mammal-derived specimen is cells.

4. The assessing method according to claim 1, wherein the mammal-derived specimen is a tissue.

5. A method for assessing a cancerous state of a mammal-derived specimen, which comprises:
(1) a first step of measuring a methylation frequency of Heparan sulfate D-glucosaminyl 3-0-sulfotransferase gene contained in the mammal-derived specimen, and
(2) a second step of determining a cancerous state of the specimen based on a difference obtained by comparing the measured methylation frequency with a control.

6. The assessing method according to claim 5, wherein Heparan sulfate D-glucosaminyl 3-0-sulfotransferase gene is Heparan sulfate D-glucosaminyl 3-0-sulfotransferase-2 gene.

7. The assessing method according to claim 1, wherein the mammal-derived specimen is cells, and the cancerous state of the specimen is a malignancy of mammal-derived cells .

8. The assessing method according to claim 6, wherein the mammal-derived specimen is cells, and the cancerous state of the specimen is a malignancy of a mammal-derived cell.

9. The assessing method according to claim 1, wherein the mammal-derived specimen is a tissue, and the cancerous state of the specimen is an amount of cancer cells existing in a mammal-derived tissue.

10. The assessing method according to claim 6, wherein the mammal-derived specimen is a tissue, and the cancerous state of the specimen is an amount of cancer cells existing in a mammal-derived tissue.

11. The assessing method according to claim 10, wherein the tissue is a breast tissue, a mammary gland tissue or a mammary gland epithelial tissue, and the cancer is breast cancer.

12. The assessing method according to claim 1 or 6, wherein the methylation frequency of a gene is a methylation frequency of cytosine in one or more nucleotide sequence(s) represented by 5' -CG-3' present in a nucleotide sequence of a promoter region or a coding region of the gene.

13. The assessing method according to claim 12, wherein the tissue is a breast tissue, a mammary gland tissue or a mammary gland epithelial tissue, and the cancer is breast cancer.

14. The assessing method according to claim 1 or 6, wherein the methylation frequency of a gene is a methylation frequency of cytosine in one or more nucleotide sequence(s) represented by 5' -CG-3' present in a nucleotide sequence of a promoter region in the gene.

15. The assessing method according to claim 1 or 6, wherein the methylation frequency of a gene is a methylation frequency of cytosine in one or more nucleotide sequence(s) represented by 5'-CG-3' present in a nucleotide sequence of a coding region of the gene.

16. The assessing method according to claim 1, wherein the methylation frequency of a gene is a methylation frequency of cytosine in one or more nucleotide sequence(s) represented by 5'-CG-3' present in the nucleotide sequence represented by SEQ ID No: 1.

17. The assessing method according to claim 16, wherein the tissue is breast tissue, mammary gland tissue or mammary gland epithelial tissue, and the cancer is breast cancer.

18. A method for assessing a cancerous state of a mammal derived specimen, which comprises :
(1) a first step of measuring an index value having the correlation with a methylation frequency of Heparan sulfate D-glucosaminyl 3-0-sulfotransferase gene contained in the mammal-derived specimen, and
(2) a second step of determining a cancerous state of the specimen based on a difference obtained by comparing the index value having the correlation with the measured methylation frequency with a control .

19. The assessing method according to claim 18, wherein Heparan sulfate D-glucosaminyl 3-0-sulfotransferase gene is Heparan sulfate D-glucosaminyl 3-0-sulfotransferase-2 gene.

20. The assessing method according to claim 18, wherein the index value having the correlation with a methylation frequency of Heparan sulfate D-glucosaminyl 3-0-sulfotransferase gene is an amount of an expression product of the Heparan sulfate D-glucosaminyl 3-0-sulfotransferase gene.

21. The assessing method according to claim 19, wherein the index value having the correlation with a methylation frequency of Heparan sulfate D-glucosaminyl 3-0-sulfotransferase gene is an amount of an expression product of the Heparan sulfate D-glucosaminyl 3-0-sulfotransferase gene.

22. The assessing method according to claim 20 or 21, wherein the amount of an expression product of Heparan sulfate D-glucosaminyl 3-0-sulfotransferase gene is an amount of a transcription product of the gene.

23. The assessing method according to claim 20 or 21, wherein the amount of an expression product of Heparan sulfate D-glucosaminyl 3-0-sulfotransferase gene is an amount of a translation product of the gene.

24. A method for searching a substance having the ability of promoting the expression of Heparan sulfate D-glucosaminyl 3-0-sulfotransferase gene, which comprises:
(1) a first step of bringing a test substance into contact with a cancer cell,
(2) a second step of measuring an amount of an expression product of 3OST gene contained in the cancer cell after the first step (1), and
(3) a third step of determining the ability of the test substance to promote the expression of Heparan sulfate D-glucosaminyl 3-0-sulfotransferase gene possessed by , based on a difference obtained by comparing the measured amount of an expression product with a control.

25. The searching method according to claim 24, wherein Heparan sulfate D-glucosaminyl 3-0-sulfotransferase gene is Heparan sulfate D-glucosaminyl 3-0-sulfotransferase-2 gene .

26. The searching method according to claim 24, wherein the cancer cell is breast cancer cell.

27. The searching method according to claim 25, wherein the cancer cell is breast cancer cell.

28. An anti-cancer agent, which comprises a substance having the ability found by the searching method of claim 24 as an active ingredient, wherein the active ingredient is formulated into a pharmaceutically acceptable carrier.

29. An anti-cancer agent, which comprises a nucleic acid comprising a nucleotide sequence encoding an amino acid sequence of Heparan sulfate D-glucosaminyl 3-0-sulfotransferase as an active ingredient, wherein the active ingredient is formulated into a pharmaceutically acceptable carrier.

30. use of methylated Heparan sulfate D-glucosaminyl 3-0-sulfotransferase gene as a cancer marker.

31. The use according to claim 30, wherein the cancer marker is a breast cancer marker.

32. use of a methylated Heparan sulfate D-glucosaminyl 3-0-sulfotransferase-2 gene as a cancer marker.

33. The use according to claim 32, wherein the cancer marker is a breast cancer marker.

34. A method for inhibiting canceration, which comprises a step of administering a substance which reduces a methylation frequency of Heparan sulfate D-glucosaminyl 3-0-sulfotransferase gene, to cells in a body of a mammal which can be diagnosed as a cancer.

35. The canceration inhibiting method according to claim 34, wherein Heparan sulfate D-glucosaminyl 3-0-sulfotransferase gene is a Heparan sulfate D-glucosaminyl 3-0-sulfotransferase-2 gene.

36. The canceration inhibiting method according to claim 35, wherein the cancer is breast cancer .

37. The assessing method according to claim 1, wherein the mammal-derived specimen is blood derived from a human being who is under 55 years old.
